# EUROPEAN PATENT SPECIFICATION

(11) **EP 3 462 915 B1**
(45) Date of publication and mention of the grant of the patent: **14.04.2021**
(21) Application number: 16903371.9
(22) Date of filing: 01.06.2016
(51) Int. Cl.: A23L 31/15, A23L 33/16, A23L 33/165, C12P 1/02, C12N 1/14, A23K 10/16, A23K 20/20, A23L 11/00, A23L 31/00

(54) **PROCESS FOR FORMING IRON ENRICHED NUTRITIONAL PRODUCTS**
VERFAHREN ZUR HERSTELLUNG VON MIT EISEN ANGEREICHERTEM ERNÄHRUNGSPRODUKTEN
PROCÉDÉ DE FORMATION DE PRODUITS NUTRITIONNELS ENRICHIS EN FER

(43) Date of publication of application: 10.04.2019
(73) Proprietor: Cura Global Health (BVI) Limited, Road Town, Tortola (VG)
(72) Inventor: WICKING, J., Bruce, Ames, IA 50011 (US); BIAN, Yilin, Ames, IA 50011 (US)
(74) Representative: Crease, Devanand John
(86) International application number: PCT/AU2016/000188
(87) International publication number: WO 2017/205890

(56) References cited:
- WO-A1-2014/040122
- CN-A- 1 227 869
- US-A1- 2009 124 572
- US-A1- 2015 250 839
- DAS RATUL KUMAR ET AL: "Effects of Different Metallic Nanoparticles on Germination and Morphology of the FungusRhizopus oryzae1526 and Changes in the Production of Fumaric Acid", BIONANOSCIENCE, SPRINGER US, BOSTON, vol. 5, no. 4, 11 November 2015 (2015-11-11), pages 217-226, XP035964239, ISSN: 2191-1630, DOI: 10.1007/S12668-015-0183-8 [retrieved on 2015-11-11]
- M. M. W. ETSCHMANN ET AL: "Improving 2-phenylethanol and 6-pentyl- [alpha] -pyrone production with fungi by microparticle-enhanced cultivation (MPEC) : Microparticle-enhanced cultivation for flavour production in fungi", YEAST, 1 July 2014 (2014-07-01), pages n/a-n/a, XP055546241, GB ISSN: 0749-503X, DOI: 10.1002/yea.3022
- DATABASE BIOSIS [Online] BIOSCIENCES INFORMATION SERVICE, PHILADELPHIA, PA, US; 1978, BARTHAKUR H P: "SOLUBILIZATION OF RELATIVELY INSOLUBLE PHOSPHATE BY SOME FUNGI ISOLATED FROM THE RHIZOSPHERE OF RICE", XP002788277, Database accession no. PREV197967044850 & INDIAN JOURNAL OF AGRICULTURAL SCIENCES, vol. 48, no. 12, 1978, pages 762-766, ISSN: 0019-5022
- KOSMAN, D.: 'Molecular mechanisms of iron uptake in fungi' MOLECULAR MICROBIOLOGY vol. 47, 2003, pages 1185 - 1197, XP055442938
- WINKELMANN, G.: 'Ecology of siderophores with special reference to the fungi' BIOMETALS vol. 20, no. 3-4, 2007, pages 379 - 392, XP019498341

## Description

### Technical Field

The technology relates to use of filamentous fungi to chelate insoluble forms of iron to produce naturally enriched iron supplements, food fortificants and iron rich food for direct human and animal consumption.

### Background

Based on the Sixth Report on the World Nutrition Situation by the United Nations (United Nations Standing Committee on Nutrition. Progress in Nutrition, Report 6. ISSN 1564-3786) in 16/33 countries that have been repeatedly surveyed, anemia in non-pregnant women has been worsening as compared to vitamin A deficiency. The report has also stated that "there is no escaping the urgent need to widely increase the intakes of bioavailable iron, and widespread fortification is likely to be part of the solution".

Iron nutrient is classified as either heme or nonheme form. Heme iron is typically derived from meat. Depending on an individual's iron stores, 15-35% of heme iron is absorbed. Nonheme iron is the more common form in all food sources, including vegetables, fruit, grain and meat. The absorption rate of non-heme iron ranges 2-20%. The most long-term effective method to combat anemia in the developing countries is to fortify stable foods, such as cereals, flour and infant foods. The iron compounds currently used in such applications are listed by the World Health Organization. Among them, elemental iron (Fe), ferric pyrophosphate (FePP) and ferric orthophosphate (FeOP) are used due to their non-reactive nature (Hu, B. A study on elemental irons and iron compounds for food fortification. 18th International Congress of Nutrition), but the absorption by humans of these insoluble forms of iron are usually lower than that of the more soluble form, such as ferrous sulfate (FeSO4).

Another form of iron can be characterized as a natural chelated iron by microorganisms, such as iron enriched yeast and fungi (Yuan, Y., et al. Construction of a high-biomass, iron-enriched yeast strain and study on distribution of iron in the cells of Saccharomyces cerevisiae. 2004. Biotchnology Letters, 26: 311-315; PCT/AU2013/001028). Filamentous fungi have the ability to chelate a high level iron from the soluble inorganic compounds. This chelated iron has a slow release characteristic and is as equally absorbable as ferrous sulfate in humans.

The present inventors have developed a process to produce iron enriched nutritional products using insoluble iron materials.

### Summary

There is provided a process for forming a nutritional supplement containing iron, the process comprising:
providing a culture medium containing insoluble iron selected from elemental iron powders, atomized iron, electrolytic iron, H-reduced iron, CO-reduced iron, carbonyl iron, ferric pyrophosphate, and ferric orthophosphate having from 0.1 grams to up to 10 grams elemental iron per liter of culture medium; culturing filamentous fungi *A. oryzae* (*A. o.*) in the culture medium to accumulate iron in the filamentous fungi as metabolizable organic iron; and harvesting the filamentous fungi to obtain a nutritional supplement containing fungal biomass having at least 100 mg/kg organic iron in the fungal biomass.

In one embodiment the culture medium is fungal growth media to assist growth and accumulation of iron by the filamentous fungi during culture. Culture media or nutrients may be provided to assist in growth of the filamentous fungi. Examples include yeast extract, ammonium salts, urea, and potassium phosphorus.

In one embodiment the culture medium can be obtained from an agricultural by-product such as waste derived from corn, wheat, sugar beet, cane sugar, soybean, stillage and solid waste from alcohol production. Examples of such products are sugar cane and beet pulps, soybean hull, soybean process whey, wheat hull, spent grain and stillage. In one embodiment the agricultural by-product is condensed corn soluble (Syrup), corn, wheat and soybean process by-products. More preferably, the agricultural by-product is Syrup.

In one embodiment the culture medium can be obtained from a food processing by-product such as corn steeping liquor, corn stillage, soybean whey, sugar cane and beet molasses, soybean hull and wheat bran and wheat hull.

The insoluble iron includes elemental iron powders which include atomized iron, electrolytic iron, H-reduced iron, CO-reduced iron and carbonyl iron, ferric pyrophosphate, ferric orthophosphate.

In one embodiment the quantity of the insoluble iron added to the culture media ranges from about 1 gram per liter of media to up to about 3 grams per liter.

The filamentous fungi may be cultured in any suitable environment such as fermentation vessels used in both solid and liquid fermentations.

Culture of the filamentous fungi may be carried out at room temperature or elevated temperatures such as 25 to 55°C.

The filamentous fungi can be harvested by any suitable means. Examples include filtration, such as filter press, belt press; centrifugation, such as decanter, drying, such as rotary drier, steam drier. The drying temperature is typically lower than about 90°C to avoid any unwanted heat damage of the product.

In an embodiment the process further includes:
removing unaccumulated insoluble iron from the culture.

In one embodiment the nutritional supplement can contain at least about 500 mg/kg iron.

In one embodiment the nutritional supplement can contain at least about 1000 mg/kg iron.

In one embodiment the nutritional supplement can contain at least about 5000 mg/kg iron.

In one embodiment the nutritional supplement contains from about 5000 to about 150000 mg/kg iron. The iron content can be higher than 150000 mg/kg, but the yield of fungi biomass may be reduced and may not be economical in practice. The harvested filamentous fungi may be further processed to form the nutritional supplement containing iron. Further processing may include separating, crushing, grinding, fractionation, extraction, washing with cold and hot water to remove excess salts, or mild acid with pH of 2 or alkaline wash with pH of 9-10 to remove other soluble compounds.

There is also provided a nutritional supplement containing iron produced by the process described.

The nutritional supplement containing the elevated level of the mineral may be formulated as a powder, solution, drink, capsule, tablet, caplet. The fungal biomass containing iron can be processed to form powder, flake, and extruded forms which can be added to food and used as a food fortification ingredient.

The nutritional supplement may be formulated for human or animal use.

There is also provided a food product containing the nutritional supplement produced according to the process described.

Examples of fermented food products that can be supplemented with iron include miso, tempeh, soy sauce, fermented rice drinks and fermented soybean drinks.

Examples of fortified food includes, but is not limited to, condiments, salt, infant formula, breakfast cereals, wheat flour, corn flour and bean flour.

Throughout this specification, unless the context requires otherwise, the word "comprise", or variations such as "comprises" or "comprising", will be understood to imply the inclusion of a stated element, integer or step, or group of elements, integers or steps, but not the exclusion of any other element, integer or step, or group of elements, integers or steps.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed before the priority date of each claim of this specification.

In order that the present technology may be more clearly understood, preferred embodiments will be described with reference to the following drawings and examples.

### Brief Description of the Drawings

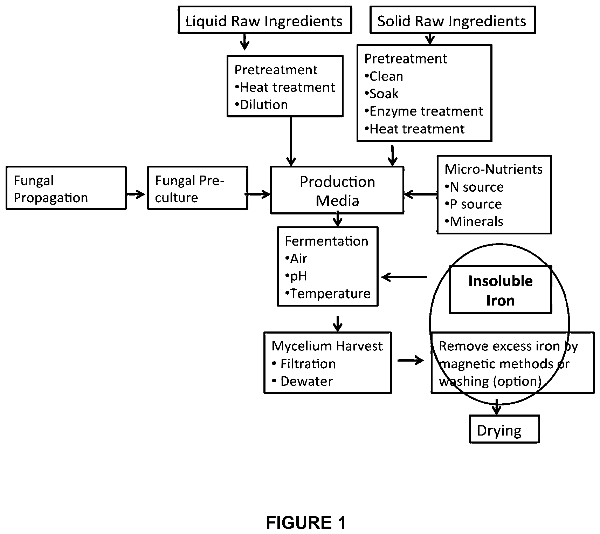

Figure 1 shows basic steps in the production of all natural iron rich fungal products from insoluble iron in liquid fermentation.

### Description of Embodiments

### Process

General steps of an embodiment are set out in Figure 1.

### Fungal strains

*Aspergillus oryzae (A.o.)* or *Aspergillus niger (A.n.)* and *Rhizopus orligosporus (R.o)* or *Rhizopus oryzae (R.oz)* have the ability to chelate insoluble forms of iron from natural sources such as soil or in controlled solid or liquid fermentations containing high concentrations of the insoluble iron.

Strains of *Aspergillus oryzae* used were the same strains that are approved and employed commercially for soy sauce and miso manufacture, including *Aspergillus oryzae* 2355 and 40151 from Chinese Center of Industrial Culture Collection (CICC); *Aspergillus oryzae* 22787 from American Type Culture Collection (ATCC) and *Aspergillus niger* var. 2206 and 10557 for citric acid production from CICC and *Aspergillus niger* 66876 for phytase production from ATCC.

### Fungal culture

Stains of *Aspergillus oryzae, Aspergillus niger, Rhizopus orligosporus (R.o)* or *Rhizopus oryzae* were cultured and maintained in media composed of ground whole corn, wheat bran, soybean hulls, molasses of beet, cane and fruits juice process by-product, and any other food process by-product consist of starch, sugar and protein. Such raw materials can be pretreated by enzymes, including amylases, gluco-amylases, phytase and protease. The insoluble iron compounds that can be added during the preparation of the growth media may include elemental iron powder, ferric pyrophosphate, ferric orthophosphate or iron oxides. The quantity of the iron powder ranges from about 1 gram per liter of media to up to 3 grams per liter. If there is any residue of iron powder that had not been utilized by fungi; a magnetic mechanism recovery system can be used to remove the iron if desired. Ferric pyrophosphate and orthophosphate residues may also be removed by filtration if desired. Insoluble forms of iron that are used in the process should be suitable for human consumption.

Fungal spores were prepared by inoculating a solid media, such as cooked rice, soybean, and sorghum and the combination of them with moisture of 40-70%. In 2-3 weeks, the spores germinated and were ready to be collected. The fungal spores were collected into sterilized distill water. The pre-cultures fermenters were prepared with 1-10% volume of the final production fermenters. The media for the pre-cultures can be the same as the production media as described above. Incubation for 18-28 hours of pre-culture fermentation time is suitable to generate healthy pre-cultures after the spores were introduced into the pre-culture media. The pre-culture is added to the production fermenter and fungus is allowed to grow to produce the desired fungal mass containing iron.

### Apparatus

Large scale fermentation can be carried out in any suitable fermentation vessel or apparatus. For the iron enriched biomass production, the fermentation is preferably carried out under aerobic conditions for 48-72 hours. Sterilized or filtered air can be pumped into the fermenter at 0.5 to 1.0 vvm during the fermentation period to improve growth and yield. The culture is preferably agitated or stirred during fermentation. The combination of air, agitation and design of the fermentation vessel is well understood for commercial microbial culture.

### Fungal fermentation

Fermentation can be carried out for 24-72 hours or until cell autolysis begins at a temperature of 28-35°C. A temperature of 28-30°C has been found to be suitable. It will be appreciated that incubation times and temperature may vary depending on the fungus type and strain used.

Depending on the nutritional profile of the raw materials, other nutrients may be needed to supplement the growth media for an aerated fungal fermentation. These nutrients may include organic and inorganic nitrogen sources, phosphors source and micro minerals.

### Production of iron enriched fungal products as an iron supplement

Fungi, including filamentous fungi, have the ability to further uptake relatively bio-unavailable and strongly cytotoxic iron. It should be noted that, direct supplementation of soluble inorganic iron salt in human diets can result in a cytotoxic reaction. Therefore, using fungi to uptake insoluble iron and transform it to an organic form may reduce the side effects of the direct consumption of iron salts.

The insoluble iron can be added during the fermentation. The common choice of the insoluble iron includes elemental iron powder, ferric pyrophosphate, ferric orthophosphate or iron oxides. To increase the level of iron in the fungal product, insoluble iron can be incrementally fed during the fermentation. The dosing of iron depends on the type of iron used but the dosing level needs to not compromise the growth of fungi. After harvest, the fungal mycelium can be thoroughly washed to remove excess iron. A mild acid, pH 2-3, wash can be effective in this regard.

### Fungal biomass harvesting

After fermentation, fungal biomass containing iron can be harvested by a dewatering machine such as a centrifuge, belt press etc. Washing with water and/or mild acid such as hydrochloric acid 0.01 M can be used to remove iron residues. The iron enriched fungal product can then be dried at 60-80°C using forced air, fluid bed dryer, etc. The final moisture of the product is preferably less than about 10%.

If there is any residue of iron powder that had not been utilized by fungi; a magnetic mechanism recovery system can be used to remove the iron if desired. Ferric pyrophosphate and orthophosphate residues may also be removed by filtration if desired.

### Formulation

For example, the nutritional supplement may be formulated for oral delivery. Nonlimiting examples of particular formulation types include tablets, capsules, caplets, powders, granules, ampoules, vials, ready-to-use solutions or suspensions, drinks, and lyophilized materials. The solid formulations such as the tablets or capsules may contain any number of suitable acceptable excipients or carriers. Food application may include powder, flake or extruded forms, or/and blended with other minerals, vitamins and food ingredients.

### Products

The nutritional supplement comprises fungal biomass having at least about 100 mg/kg iron. The nutritional supplement typically contains from about 500 to about 150000 mg/kg iron, or from about 5000 to about 150000 mg/kg iron. The supplement can have at least about 100, 200, 300, 400, 500, 600, 700, 800, 900, 1000, 1100, 1200, 1300, 1400, 1500, 1600, 1700, 1800, 1900, 2000, 2100, 2200, 2300, 2400, 2500, 2600, 2700, 2800, 2900, 3000, 3500, 4000, 4500, 5000, 5500, 6000, 6500, 7000, 7500, 8000, 8500, 9000, 9500, 10000, 11000, 12000, 13000, 14000, 15000, 16000, 17000, 18000, 19000, 20000, 21000, 22000, 23000, 24000, 25000, 26000, 27000, 28000, 29000, 30000, 31000, 32000, 33000, 34000, 35000, 36000, 37000, 38000, 39000, 40000, 41000, 42000, 43000, 44000, 45000, 46000, 47000, 48000, 49000, 50000, 60000, 70000, 80000, 90000, 100000, 110000, 120000, 130000, 140000, 150000 or more mg/kg iron.

The nutritional supplement containing the elevated level of the mineral may be formulated as a powder, solution, drink, capsule, tablet, caplet. The biomass can be processed to form powder, flake, and extruded forms which can be added to food and used as a food fortification ingredient. The fortification of food includes, but is not limited to condiments, salt, baby formula, breakfast cereals and flours of wheat, corn and beans.

An advantage of the nutritional supplement is that it contains natural organic iron derived from fermentation by the fungi. The nutritional supplement may be formulated to further contain phytase and other enzymes naturally produced by the filamentous fungi.

The nutritional supplement can be formulated for human or animal use.

### Liquid Fungal Fermentation: Production of all natural iron rich fungal products

The base of the fermentation media consisted of but was not limited to corn, wheat, soybean and rice as the carbon sources. The by-products of agricultural processing and food processing may also be used as both carbon and nitrogen sources. Depending on the nutritional profile of the raw materials, other nutrients may be needed to supplement the growth media during an aerated fungal fermentation. These nutrients may include organic and inorganic nitrogen sources, phosphors source and micro minerals.

Detailed steps of the process are described in Figure 1. All fungi, *A.o.*, *A.n.*, *R.o and R.oz* are suitable for liquid fermentation.

The insoluble iron compounds that can be added during the preparation of the growth media may include elemental iron powder, ferric pyrophosphate, ferric orthophosphate or iron oxides. The quantity of the iron powder ranges from 1 gram per liter of media to up to 3 grams per liter. If there is any residue of iron powder that had not been utilized by fungi; a magnetic mechanism recovery system can be used to remove the iron if desired. Ferric pyrophosphate and orthophosphate residues may also be removed by filtration if desired. Insoluble forms of iron that are used in the process should be suitable for human consumption.

### Solid Fungal Fermentation: Production of all natural iron enriched food for direct consumption or food fortification

### Natural Iron Enriched Tempeh

The basic fermentation techniques of making tempeh using *R.o.* and/or *R.oz.* are described in various ways and range from more traditional methods to production in more controlled environments. Insoluble forms of iron compound can be added during the soybean cooking stage. Once the fungi has been grown during the fermentation, partially solubilizing and incorporating the iron into the cell structure, as shown in Figure 1. The advantage of using the insoluble iron versus the soluble iron, such as ferrous sulfate and EDTA-iron, is that the insoluble iron upon biological conversion is unlikely to cause flavor and color changes during the fermentation and the subsequent storage and cooking.

### Natural Iron Enriched Miso

When added to the making of miso, the insoluble iron will endure a prolonged fermentation process and will gradually be solubilized by *A.o.,* or other microorganisms during the aging of miso, to form organically chelated iron compounds. Such naturally derived soluble iron is highly bioavailable without the high cost of EDTA-iron which is associated with special market efforts, such as EDTA-iron fortified soy sauce.

### EXAMPLE 1

Elemental iron, including atomized, H-reduced and electrolytic iron can be added to a liquid fermentation to produce iron enriched A.o.. Using corn and wheat bran as the base media, atomized iron was added during the media preparation and A.o. inoculation. After a 48-hour aerated fermentation, the iron content in the A.o. biomass was significantly increased. A magnet was used to remove excess iron powder and 23.5% excess iron powder was removed from the fungal biomass when Sample C was compared to Sample B (Table 1).

**Table 1. Iron Content in A.o. Biomass When Grown in Atomized Iron Enriched Liquid Media**

| **Sample (Fermentation volume)** | **¹Atomized iron, mg/L** | **Fe in *A. oryzae* biomass mg/g** | **% Fe Uptake by *A.oryzae*** |
|---|---|---|---|
| A (300 ml volume) | 1000 | 16.3 | 33.0% |
| B (5000 ml volume) | 1000 | 15.3 | 31.9% |
| C (5000 ml volume) | ²1000 | 11.7 | 24.3% |

| | | | |
|---|---|---|---|
| 1. Atomized iron added to liquid growth media. 2. Sample C was processed from Sample B by using a magnet to remove excess iron powder. | | | |

### EXAMPLE 2

Insoluble ferric pyrophosphate (FePP) has been approved for use as a food additive. Using A.o. in a liquid fermentation with the addition of FePP resulted in an increased iron content in the fungal biomass. The chelating of FePP by *A.o.* was very strong as demonstrated by a high iron content. All of the added iron in FePP was chelated to the fungal biomass. The yield of fungal biomass was also 25.2% higher with FePP enriched media as compared to the one enriched with elemental iron powder (Example 1 above). The increase in biomass yield might be explained by the phosphate component in FePP. In Table 2, sample C was prepared from Sample B by washing B with deionized water. The washing removed about 30% of iron from the fungal biomass, but the iron content in sample C was still over 7% (70.7 mg/g).

**Table 2. Iron Content in A.o. Biomass When Grow in Ferric Pyrophosphate Enriched Liquid Media**

| **Sample** | **FePP, mg/L (as Fe based)** | **Fe in *A. oryzae* biomass, mg/g** | **% Fe Uptake by *A. oryzae*** |
|---|---|---|---|
| A | 1200 | 30.6 | 100% |
| B | 2000 | 106.3 | 100% |
| c | ²2000 | 70.7 | 71% |

### EXAMPLE 3

An *in vitro* digestibility and Caco-2 cell study (Au, A.P. and Reddy, M.B., Caco-2 cell can be used to assess human iron bioavailability from a semi purified meal. 2000. J. Nutr. 130(5):1329-1334) was conducted on the bioavailability of A.o. fermented with FePP enriched media (FePP-Ao) and elemental iron enriched media (Fe-Ao) as compared to the insoluble FePP and elemental iron powder. Using ferrous sulfate (FeSO4) as 100% soluble, the solubility of other iron containing products is listed in Table 3. Both FePP-Ao and Fe-Ao had higher digestibility than the chemical counterparts. But the ferritin formation in the Caco-2 cell for the FePP-Ao was lower than the FePP. It is likely that the chelated FePP-Ao had a tighter bonding between iron to Ao and formed larger molecular weight organic compounds. Since the Caco-2 cell test is used primarily on inorganic iron compounds, it may not produce a good correlation between ferritin formation to the *in vivo* testing in humans. The Fe-Ao had higher ferritin response than the iron powder.

The atomized iron powder is widely used in food fortification, the use of Fe-Ao may improve the iron bioavailability.

**Table 3. In vitro Solubility of Iron Enriched A.o. as Compare to the Chemical Iron Products**

| **Products** | **Digestibility As % of FeSO₄** | **Ferritin in CaCO₂ As % of FeSO₄** |
|---|---|---|
| FeSO₄ | 100 | 100 |
| FePP | 47 | 100 |
| FePP-Ao | 77 | 23 |
| Fe Powder | 62 | 60 |
| Fe-Ao | 100 | 73 |

### EXAMPLE 4

The formation of a multi-mineral A.o. biomass using FePP as the iron source plus other mineral compounds has also been achieved. The uptake of all minerals by the fungi could range from 80% up to 100% (Table 4). The mineral concentration in Table 4 demonstrates a particular mineral combination and concentration. The resulting multi-mineral rich A.o. biomass, when used in a 0.5 gram dose provided up to 99% of Recommended Daily Requirement (RDA) of the minerals recommended by the Food and Drug Administration (FDA) of the US (Table 5). The type and concentration of each mineral can be tailor made to suit any mineral requirement in multi-vitamins or functional foods.

**Table 4. Multi-Mineral Contents in A.o. Biomass When Grown in Ferric Pyrophosphate and Other Mineral Enriched Liquid Media**

| **Inorganic Mineral** | **Inorganic Mineral in Media, as salt (as element)** | **Organic Mineral in A.o. biomass ppm** | **% Mineral Uptake by fungi** |
|---|---|---|---|
| Ferric pyrophosphate | 2.2 (0.44 g Fe/L) | 24500 | 100.00 |
| Zinc sulfate (ZnSO₄·7H₂O) | 1.5 (0.34 g Zn/L) | 18200 | 98.63 |
| Manganese sulfate (MnSO₄·H₂O) | 0.3 (0.10 g Mn/L) | 4170 | 80.48 |
| Cupric sulfate (CuSO₄·6h₂O) | 0.2 (0.05 g Cu/L) | 2805 | 100.00 |
| Chromium chloride (CrCl₃·6H₂O) | 0.016 (3.0 mg/L) | 147 | 89.44 |
| Sodium selenite (Na₂SeO₃) | 0.002 (0.9 mg/L) | 42.3 | 86.00 |

**Table 5. Percentage of RDA in a 0.5 gram dose of A.o. Biomass Dried Powder**

| **Mineral** | **FDA RDA (mg/d)** | **Concentration (mg/kg)** | **% RDA/0.5 g** |
|---|---|---|---|
| Iron | 18 | 23200 | **64** |
| Zinc | 15 | 17200 | **57** |
| Selenium | 0.045 | 40.1 | **44** |
| Chromium | 0.12 | 139 | **58** |
| Copper | 2 | 2700 | **68** |
| Manganese | 2 | 3950 | **99** |

### EXAMPLE 5

Tempeh and Miso are two of the most popular Asian foods that use or in part use *R.o and*/*or R.oz and A.o.* respectively in solid fermentation of soybeans. Because the fungi are able to chelate insoluble iron and transform the iron to an organic form with increased bioavailability, it has been found by the present inventors that iron powder or FePP can be used to enrich the soybean iron content before the solid fermentation. The resulting tempeh or miso had organically enriched iron for human consumption. With 10 mg elemental iron per 100 grams of cooked soybean prior to tempeh fermentation, the growth of *R. oryzae* was the same as the process without added iron. The appearance of the finished tempeh was similar and there were no perceived sensory or taste differences.

Because use of iron powder is more economical than FePP as a food fortificant, the use of iron powder to enrich tempeh or miso may provide an improved bioavailable iron source for the population of countries consuming these foods. Ferrous sulfate and sodium iron EDTA have been studied in the tempeh fermentation but do not result in a suitable commercial product. Ferrous sulfate may either result in color changes in tempeh or changes in other food when cooked together. Sodium iron EDTA is much more expensive for use as a stable food fortificant so may not be commercially viable in many situations.

This technology relates to methods for the use of fungi, *Aspergillus oryzae (A.o.)* to chelate insoluble forms of iron compounds via either liquid fermentation or solid fermentation to produce naturally mineral enriched fungal biomass for use as iron supplements, food fortificants and iron rich food for direct human or animal consumption.

It will be appreciated by persons skilled in the art that numerous variations and/or modifications may be made to the invention as shown in the specific embodiments without departing from the scope of technology as broadly described. The present embodiments are, therefore, to be considered in all respects as illustrative and not restrictive.

## Claims

1. A process for forming a nutritional supplement containing iron, the process comprising:
providing a culture medium containing insoluble iron selected from elemental iron powders, atomized iron, electrolytic iron, H-reduced iron, CO-reduced iron, carbonyl iron, ferric pyrophosphate, and ferric orthophosphate having from 0.1 grams to up to 10 grams elemental iron per liter of culture medium;
culturing filamentous fungi *Aspergillus oryzae (A.o.)* in the culture medium to accumulate iron in the filamentous fungi as metabolizable organic iron; and
harvesting the filamentous fungi to obtain a nutritional supplement containing fungal biomass having at least 100 mg/kg organic iron in the fungal biomass.

2. The process according to claim 1 wherein the insoluble iron is atomized iron, or ferric pyrophosphate.

3. The process according to claim 1 or 2 wherein the insoluble iron is added to the culture media from 1 gram per liter of media to up to 3 grams per liter.

4. The process according to any one of claims 1 to 3 further including:
removing unaccumulated insoluble iron from the harvested biomass.

5. The process according to any one of claims 1 to 4 wherein the nutritional supplement contains at least 1000 mg/kg organic iron.

6. The process according to claim 5 wherein the nutritional supplement contains 5000 to 150000 mg/kg organic iron.

7. The process according to any one of claims 1 to 6 wherein the insoluble iron is ferric pyrophosphate and the process further comprises adding one or more minerals selected from the group consisting of zinc sulfate, manganese sulfate, cupric sulfate, chromium chloride, and sodium selenite to the culture medium, culturing the filamentous fungi to accumulate the one or more minerals in the filamentous fungi as metabolizable organic minerals, and harvesting the fungal biomass to form a nutritional supplement having at least 100 mg/kg organic iron and at least one of organic zinc, manganese, copper, chromium, or selenium in the fungal biomass.

8. The process according to claim 7 comprising adding the minerals zinc sulfate, manganese sulfate, cupric sulfate, chromium chloride, and sodium selenite to the culture medium, culturing the filamentous fungi to accumulate the minerals in the filamentous fungi as metabolizable organic minerals, and harvesting the fungal biomass to form a nutritional supplement having at least 100 mg/kg organic iron and organic zinc, manganese, copper, chromium, and selenium in the fungal biomass.

## Patentansprüche

1. Vorgang zum Ausbilden eines Nahrungsergänzungsmittels, das Eisen enthält, wobei der Vorgang umfasst:
Bereitstellen eines Kulturmediums, das unlösliches Eisen enthält, das aus elementaren Eisenpulvern, zerstäubtem Eisen, elektrolytischem Eisen, H-reduziertem Eisen, CO-reduziertem Eisen, Carbonyleisen, Eisen(III)-pyrophosphat und Eisen(III)-orthophosphat, das 0,1 Gramm bis zu 10 Gramm elementares Eisen pro Liter Kulturmedium aufweist, ausgewählt ist;
Kultivieren von Fadenpilzen *Aspergillus oryzae (A.o.)* in dem Kulturmedium, um Eisen in den Fadenpilzen als metabolisierbares organisches Eisen anzusammeln; und
Ernten der Fadenpilze, um ein Nahrungsergänzungsmittel zu erhalten, das Pilzbiomasse, die wenigstens 100 mg/kg organisches Eisen in der Pilzbiomasse aufweist, enthält.

2. Vorgang nach Anspruch 1, wobei das unlösliche Eisen zerstäubtes Eisen oder Eisen(III)-pyrophosphat ist.

3. Vorgang nach Anspruch 1 oder 2, wobei das unlösliche Eisen dem Kulturmedium von 1 Gramm pro Liter Medium bis zu 3 Gramm pro Liter zugesetzt wird.

4. Vorgang nach einem der Ansprüche 1 bis 3, der ferner beinhaltet:
Entfernen von nicht angesammeltem unlöslichem Eisen aus der geernteten Biomasse.

5. Vorgang nach einem der Ansprüche 1 bis 4, wobei das Nahrungsergänzungsmittel wenigstens 1000 mg/kg organisches Eisen enthält.

6. Vorgang nach Anspruch 5, wobei das Nahrungsergänzungsmittel 5000 bis 150000 mg/kg organisches Eisen enthält.

7. Vorgang nach einem der Ansprüche 1 bis 6, wobei das unlösliche Eisen Eisen(III)-pyrophosphat ist und der Vorgang ferner das Zusetzen eines oder mehrerer Mineralien, die aus der Gruppe ausgewählt sind, die aus Zinksulfat, Mangansulfat, Kupfer(II)-sulfat, Chromchlorid und Natriumselenit besteht, zu dem Kulturmedium, das Kultivieren der Fadenpilze, um das eine oder die mehreren Mineralien in den Fadenpilzen als metabolisierbare organische Mineralien anzusammeln, und das Ernten der Pilzbiomasse umfasst, um ein Nahrungsergänzungsmittel, das wenigstens 100 mg/kg organisches Eisen und organisches Zink, Mangan, Kupfer, Chrom und/oder Selen in der Pilzbiomasse aufweist, auszubilden.

8. Vorgang nach Anspruch 7, der das Zusetzen der Mineralien Zinksulfat, Mangansulfat, Kupfer(II)-sulfat, Chromchlorid und Natriumselenit zu dem Kulturmedium, das Kultivieren der Fadenpilze, um die Mineralien in den Fadenpilzen als metabolisierbare organische Mineralien anzusammeln, und das Ernten der Pilzbiomasse umfasst, um ein Nahrungsergänzungsmittel, das wenigstens 100 mg/kg organisches Eisen und organisches Zink, Mangan, Kupfer, Chrom und Selen in der Pilzbiomasse aufweist, auszubilden.

## Revendications

1. Procédé de formation d'un complément nutritionnel contenant du fer, le procédé comprenant :
la fourniture d'un milieu de culture contenant du fer insoluble choisi parmi les poudres de fer élémentaire, le fer atomisé, le fer électrolytique, le fer réduit par hydrogène, le fer réduit par carbone monoxyde, le fer carbonyle, le pyrophosphate ferrique et l'orthophosphate ferrique ayant de 0,1 gramme à jusqu'à 10 grammes de fer élémentaire par litre de milieu de culture ;
la culture de champignons filamenteux *Aspergillus oryzae (A.o.)* dans le milieu de culture pour accumuler du fer dans les champignons filamenteux sous forme de fer organique métabolisable ; et
la récolte des champignons filamenteux pour obtenir un complément nutritionnel contenant de la biomasse fongique ayant au moins 100 mg/kg de fer organique dans la biomasse fongique.

2. Procédé selon la revendication 1, dans lequel le fer insoluble est du fer atomisé ou du pyrophosphate ferrique.

3. Procédé selon la revendication 1 ou 2, dans lequel le fer insoluble est ajouté au milieu de culture à partir de 1 gramme par litre de milieu jusqu'à 3 grammes par litre.

4. Procédé selon l'une quelconque des revendications 1 à 3, comportant :
l'élimination du fer insoluble non accumulé de la biomasse récoltée.

5. Procédé selon l'une quelconque des revendications 1 à 4, dans lequel le complément nutritionnel contient au moins 1000 mg/kg de fer organique.

6. Procédé selon la revendication 5, dans lequel le complément nutritionnel contient de 5 000 à 150 000 mg/kg de fer organique.

7. Procédé selon l'une quelconque des revendications 1 à 6, dans lequel le fer insoluble est le pyrophosphate ferrique et le procédé comprend en outre l'ajout d'un ou plusieurs minéraux choisis dans le groupe constitué du sulfate de zinc, du sulfate de manganèse, du sulfate de cuivre, du chlorure de chrome et du sélénite de sodium au milieu de culture, la culture des champignons filamenteux pour accumuler le ou les minéraux dans les champignons filamenteux en tant que minéraux organiques métabolisables, et la récolte de la biomasse fongique pour former un complément nutritionnel contenant au moins 100 mg/kg de fer organique et du zinc et/ou du manganèse et/ou du cuivre et/ou du chrome et/ou du sélénium organiques dans la biomasse fongique.

8. Procédé selon la revendication 7, comprenant l'ajout des minéraux de sulfate de zinc, de sulfate de manganèse, de sulfate de cuivre, de chlorure de chrome et de sélénite de sodium au milieu de culture, la culture des champignons filamenteux pour accumuler les minéraux dans les champignons filamenteux en tant que minéraux organiques métabolisables, et la récolte des la biomasse fongique pour former un complément nutritionnel contenant au moins 100 mg/kg de fer organique et de zinc, de manganèse, de cuivre, de chrome et de sélénium organiques dans la biomasse fongique.
